# EUROPEAN PATENT APPLICATION

(11) **EP 4 825 421 A1**
(43) Date of publication of application: **21.10.2026**
(21) Application number: 25845846.2
(22) Date of filing: 09.12.2025
(51) Int. Cl.: A61M 16/00, A61M 16/04

(54) **LARYNGEAL MASK AND USE METHOD THEREFOR**

(30) Priority: 28.02.2025 CN 202510232008; 04.11.2025 CN 202511603270
(71) Applicant: Tianjin Medis Medical Device Co., Ltd., Tianjin 300385 (CN)
(72) Inventor: ZOU, Dewei, Xiqing District, Tianjin 300385 (CN)
(74) Representative: Vogelbruch, Keang
(86) International application number: PCT/CN2025/141036
(87) International publication number: WO 2026/179299

(57) **Abstract**

The embodiment of the present invention provides a laryngeal mask and a method of using therefor, relating to the technical field of medical devices. The laryngeal mask includes a laryngeal mask body, a mask cuff, a resilient member, and a locking assembly. The mask cuff itself is elastic and is provided with a compression space and a resilient member therein, and the compression space is communicated with an openable and closable negative pressure channel, so that the mask cuff can be compressed. The negative pressure channel can be closed through the locking assembly to enable the mask cuff to maintain a compressed state, thereby reducing the volume of the laryngeal mask before insertion to facilitate insertion and reducing insertion difficulty and dependence on operation skills. After the laryngeal mask is inserted in place, the negative pressure channel is conducted again through the locking assembly. Under the action of elasticity of the mask cuff itself and the resilient member, the mask cuff expands adaptively until it fits the unique laryngeal anatomical structure of the patient, which can better adapt to the differences in cavities of different patients, thereby providing a high-sealing airtight effect at the laryngeal inlet and improving ventilation safety.

## Description

### CROSS-REFERENCE TO RELATED APPLICATION

The present invention claims priority to Chinese Patent Application No. 202511603270.6, entitled "LARYNGEAL MASK AND METHOD OF USING THEREFOR" filed with the China National Intellectual Property Administration on November 4, 2025, and Chinese Patent Application No. 202510232008.9, entitled "LARYNGEAL MASK AIRWAY" filed with the China National Intellectual Property Administration on February 28, 2025, the entire contents of which are incorporated herein by reference.

### TECHNICAL FIELD

The present invention belongs to the technical field of medical devices, and specifically relates to a laryngeal mask and a method of using therefor.

### BACKGROUND ART

In clinical anesthesia and emergency care, establishing a reliable artificial airway is crucial. As a supraglottic airway tool, the laryngeal mask has become an indispensable device because its operation is simpler than endotracheal intubation and it can effectively cope with "difficult airways."

Currently, laryngeal masks are mainly divided into two types: inflatable and non-inflatable. An inflatable laryngeal mask seals the laryngeal inlet by inflating the cuff. However, its volume is relatively large after inflation, which tends to cause excessive compression on the pharyngeal and laryngeal tissues and poses a risk of injury. Furthermore, the inflation volume relies on the empirical judgment of the physician, posing risks of air leakage due to poor sealing or excessive pressure. To avoid the disadvantages of the inflatable type, non-inflatable laryngeal masks that do not require inflation have been developed. Non-inflatable laryngeal masks are made of elastic materials and prefabricated into fixed shapes, and do not require inflation after placement.

However, to ensure support and sealing, the mask body of a non-inflatable laryngeal mask usually possesses a certain degree of rigidity, resulting in a relatively large volume that makes insertion inconvenient and requires high operational skills. Moreover, since the laryngeal mask is manufactured into a preset shape in advance, its adaptability to the cavities of different patients varies, which may lead to poor sealing and affect ventilation safety.

### SUMMARY

The objective of the present invention is to provide a laryngeal mask to solve the technical problems in the prior art that a non-inflatable laryngeal mask has a relatively large volume, is inconvenient to insert, and has varying adaptability, which may lead to poor sealing.

An embodiment of the present invention provides a laryngeal mask, including: a laryngeal mask body, a mask cuff, a resilient member, and a locking assembly, wherein
the airflow channel is formed inside the laryngeal mask body;
the mask cuff is disposed at one end of the laryngeal mask body and located at one side of the laryngeal mask body, the mask cuff is arranged along a circumferential direction of the laryngeal mask body and encloses at the one side of the laryngeal mask body to form a ventilation port communicating with the airflow channel, the mask cuff is elastic and a compression space is formed therein;
the resilient member is disposed in the compression space and connected with an inner wall of the mask cuff; and
the locking assembly has a negative pressure channel which communicates with the compression space and is openable and closable.

Optionally, a transmission channel is further formed in the laryngeal mask body; and
one end of the transmission channel is connected with the mask cuff and communicates with the compression space, and the other end is connected with the locking assembly and communicates with the negative pressure channel.

Optionally, the laryngeal mask body includes: an insertion portion and a conduction portion;
one side of the insertion portion is an installation end provided with a first through hole communicating with the airflow channel, and the mask cuff is disposed at the installation end; and
the conduction portion is of an arc-shaped structure and one end of the conduction portion is connected with the insertion portion, both the airflow channel and the transmission channel are disposed in the conduction portion, and the locking assembly is connected with the conduction portion.

Optionally, the laryngeal mask further includes an air source connector;
an air source port is formed at one end of the airflow channel away from the mask cuff and at one end of the conduction portion away from the insertion portion, and a negative pressure port is formed at one end of the transmission channel away from the mask cuff and at one end of the conduction portion away from the insertion portion;
the air source connector is connected with the air source port and configured to connect to an air source device; and
the negative pressure port is connected with the locking assembly.

Optionally, a drainage channel and an air intake channel are further disposed in the laryngeal mask body;
one end of the insertion portion away from the conduction portion is provided with a drainage port;
one end of the drainage channel and one end of the air intake channel are connected with the drainage port, and the other ends of the drainage channel and the air intake channel respectively form a drainage tube port and an air intake port at an end of the conduction portion away from the insertion portion.

Optionally, the locking assembly includes: an air pass tube and a locking member;
the air pass tube is inserted in the laryngeal mask body, and the transmission channel is disposed in the air pass tube; and
the locking member is disposed on the air pass tube, and the locking member is capable of closing the transmission channel.

Optionally, the locking member is a stop clip; and
the stop clip is disposed on the air pass tube and capable of clamping the air pass tube to close the transmission channel.

Optionally, the laryngeal mask body and the mask cuff are adhesively connected to form a sealed structure at a connection.

Optionally, the resilient member is made of a porous elastic material; and
a shape of the resilient member is adapted to the compression space.

Optionally, the resilient member is a medical sponge.

Optionally, the mask cuff is made of a polymer material.

Optionally, a Shore hardness of the material of the mask cuff is less than 40.

Optionally, the Shore hardness of the material of the mask cuff is 4 to 12.

Optionally, the mask cuff is made of medical silicone or polyurethane material.

The embodiment of the present invention further provides a method of using for a laryngeal mask applied to the laryngeal mask provided; and the method of using includes:
compressing the mask cuff and the resilient member to discharge air in the compression space;
adjusting the locking assembly to close the negative pressure channel;
applying a lubricant to the mask cuff and the end of the laryngeal mask body where the mask cuff is disposed;
inserting the laryngeal mask body into a mouth until the mask cuff reaches a preset position; and
adjusting the locking assembly to conduct the negative pressure channel.

The beneficial effects provided in this embodiment are as follows.

In the embodiment of the present invention, the mask cuff itself is elastic and is provided with a compression space and a resilient member therein, and the compression space is communicated with an openable and closable negative pressure channel, so that the mask cuff can be compressed, and the negative pressure channel can be closed through the locking assembly to enable the mask cuff to maintain a compressed state, thereby reducing the volume of the laryngeal mask before insertion to facilitate insertion and reducing insertion difficulty and dependence on operation skills. After the laryngeal mask is inserted in place, the negative pressure channel is conducted again through the locking assembly. Under the action of elasticity of the mask cuff itself and the resilient member, the mask cuff expands adaptively until it fits the unique laryngeal anatomical structure of the patient, which can better adapt to the differences in cavities of different patients, thereby providing a high-sealing airtight effect at the laryngeal inlet and improving ventilation safety.

### BRIEF DESCRIPTION OF DRAWINGS

In order to more clearly illustrate the technical solutions in the specific embodiments of the present invention or in the prior art, the following will briefly introduce the drawings required for the description of the specific embodiments or the prior art. Obviously, the drawings described below are some embodiments of the present invention, and for a person of ordinary skill in the art, other drawings can be obtained based on these drawings without inventive efforts.
FIG. 1 is a schematic structural diagram of a laryngeal mask provided by an embodiment of the present invention;
FIG. 2 is a schematic diagram of a separated structure of a laryngeal mask provided by an embodiment of the present invention;
FIG. 3 is a schematic diagram illustrating a positional relationship of an airflow channel in the laryngeal mask provided by an embodiment of the present invention;
FIG. 4 is a schematic diagram illustrating positional relationships of a transmission channel and an air intake channel in the laryngeal mask provided by an embodiment of the present invention;
FIG. 5 is a schematic structural diagram of a drainage channel in the laryngeal mask provided by an embodiment of the present invention; and
FIG. 6 is a schematic structural diagram of a locking assembly of the laryngeal mask provided by an embodiment of the present invention.

### Reference Numerals:

100, laryngeal mask body; 101, insertion portion; 102, conduction portion; 110, airflow channel; 120, transmission channel; 130, drainage channel; 140, air intake channel; 200, mask cuff; 300, resilient member; 400, locking assembly; 410, air pass tube; 420, locking member; and 500, air source connector.

### DETAILED DESCRIPTION OF EMBODIMENTS

In order to make the objectives, technical solutions, and advantages of the embodiments of the present invention clearer, the technical solutions of the embodiments of the present invention will be described clearly and completely in conjunction with the drawings in the embodiments of the present invention. Obviously, the described embodiments are part of the embodiments of the present invention, rather than all of the embodiments. Generally, the components of the embodiments of the present invention described and shown in the drawings herein can be arranged and designed in various different configurations.

Therefore, the detailed description of the embodiments of the present invention provided in the drawings is not intended to limit the scope of the claimed invention, but merely to represent selected embodiments of the present invention. Based on the embodiments in the present invention, all other embodiments obtained by a person of ordinary skill in the art without inventive efforts shall fall within the scope of protection of the present invention.

It should be noted that similar reference numerals and letters in the following drawings indicate similar items. Therefore, once an item is defined in one drawing, it does not need to be further defined and explained in the subsequent drawings.

In the description of the present invention, it should be noted that the orientation or position relationships indicated by terms "center", "upper", "lower", "left", "right", "vertical", "horizontal", "inner", "outer", etc., are based on the orientation or position relationships shown in the drawings, or the orientation or position relationships in which the product of the present invention is usually placed when used. They are only for the convenience of describing the present invention and simplifying the description, rather than indicating or implying that the referred device or element must have a specific orientation, be constructed and operated in a specific orientation, and therefore cannot be understood as a limitation to the present invention. In addition, the terms "first", "second", "third", etc., are only used for distinguishing description, and cannot be understood as indicating or implying relative importance.

Furthermore, terms such as "horizontal" and "vertical" do not require that the components be absolutely horizontal or vertical, but can be slightly inclined. For example, "horizontal" only means that its direction is more horizontal relative to "vertical", and does not mean that the structure must be completely horizontal, but can be slightly inclined.

In the description of the present invention, it should also be noted that unless otherwise clearly specified and limited, the terms "provide", "mount", "connect", and "link" should be understood in a broad sense. For example, it can be a fixed connection, a detachable connection, or an integral connection; it can be a mechanical connection, or an electrical connection; it can be a direct connection, or an indirect connection through an intermediate medium, and it can be the internal communication of two elements. For a person of ordinary skill in the art, the specific meanings of the above terms in the present invention can be understood according to specific circumstances.

The present invention will be further described in detail below through specific embodiments in conjunction with the drawings.

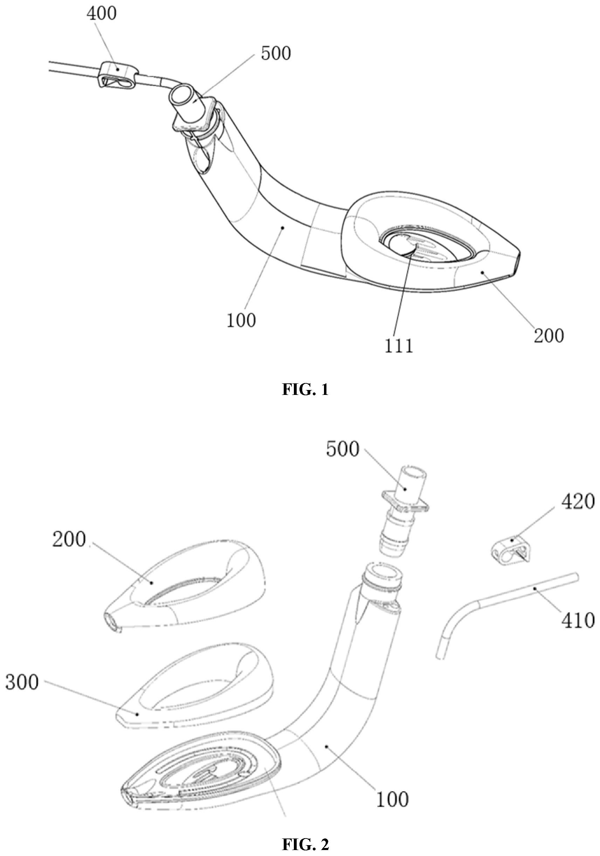

Please refer to FIG. 1 to FIG. 6. A laryngeal mask provided in this embodiment includes: a laryngeal mask body 100, a mask cuff 200, a resilient member 300, and a locking assembly 400.

An airflow channel 110 is formed inside the laryngeal mask body 100. The mask cuff 200 is disposed at one end of the laryngeal mask body 100 and located on one side of the laryngeal mask body 100. The mask cuff 200 is arranged along a circumferential direction of the laryngeal mask body 100 and encloses at the one side of the laryngeal mask body 100 to form a ventilation port communicating with the airflow channel 110. The mask cuff 200 is elastic and a compression space is formed therein. The resilient member 300 is disposed in the compression space and connected with an inner wall of the mask cuff 200. The locking assembly 400 has a negative pressure channel which communicates with the compression space and is openable and closable.

Specifically, in this embodiment, before the laryngeal mask is inserted, the mask cuff 200 is manually pressed, the mask cuff 200 is enabled to be compressed, or air in the compression space of the mask cuff 200 is extracted by using a negative pressure generating member through the negative pressure channel, so that the mask cuff 200 is actively compressed under negative pressure and its volume is significantly reduced.

Subsequently, the negative pressure channel is closed by the locking assembly 400 to close the outlet of the compression space. The mask cuff 200 maintains a compressed state, thereby reducing the volume of the laryngeal mask, actively transforming the laryngeal mask from a larger preset working state to a temporary compressed state with a smaller volume. This reduces insertion resistance and the risk of scraping surrounding tissues, thereby facilitating insertion, and further reducing insertion difficulty and dependence on operation skills.

After the laryngeal mask is inserted in place, the locking assembly 400 can be adjusted again to conduct the negative pressure channel. External air immediately enters the compression space. After losing the constraint of external pressure, the cuff 200 is driven to expand freely outward from its compressed state by the elastic restoring force of the cuff 200 itself and the elastic restoring forces of the resilient member 300, until the outer surface of the mask cuff 200 is closely fitted to the unique laryngeal anatomical contour of the patient. In this structure, the final shape of the mask cuff 200 is not a fixed preset shape, but a dynamically formed personalized sealing interface determined by the patient own anatomical structure and the characteristics of the resilient member 300. It can adapt to the differences in cavities of different patients, thereby providing a high-sealing airtight effect at the laryngeal inlet and improving ventilation safety.

In this embodiment, a transmission channel 120 is further formed in the laryngeal mask body 100.

One end of the transmission channel 120 is connected with the mask cuff 200 and communicates with the compression space, and the other end is connected with the locking assembly 400 and communicates with the negative pressure channel.

The transmission channel 120 is a dedicated channel connecting the compression space and the locking assembly 400. When the mask cuff 200 is pressed, air in the compression space can be smoothly discharged through the transmission channel 120. When the laryngeal mask is placed in place, by conducting the negative pressure channel through the locking assembly 400, external air can smoothly and unobstructedly flow back into the compression space via the transmission channel 120.

In this structure, it is ensured that the resilient member 300 can obtain recovery space in a timely and uniform manner, thereby driving the mask cuff 200 to expand smoothly until it achieves a complete and close self-adaptive fit with the laryngeal anatomical structure of the patient. This avoids incomplete recovery or local wrinkles of the mask cuff 200 caused by poor air backflow, and improves the quality of the final sealing interface and ventilation safety.

Moreover, in this embodiment, the transmission channel 120 is built inside the laryngeal mask body 100, making the overall structure of the laryngeal mask more compact and highly integrated. It avoids the risk of interference or damage that may be caused by exposed pipelines, and has stronger integrity and a more concise and intuitive operation process.

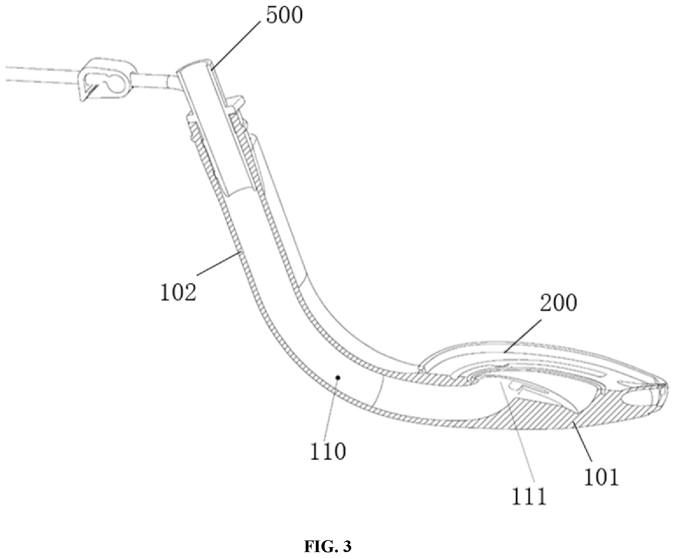

Combined with FIG. 3, in this embodiment, the laryngeal mask body 100 includes an insertion portion 101 and a conduction portion 102.

One side of the insertion portion 101 is an installation end provided with a first through hole communicating with the airflow channel 110, and the mask cuff 200 is disposed at the installation end.

The conduction portion 102 is an arc-shaped structure. One end of the conduction portion 102 is connected with the insertion portion 101. Both the airflow channel 110 and the transmission channel 120 are disposed in the conduction portion 102, and the locking assembly 400 is connected with the conduction portion 102.

The insertion portion 101 is configured to install the mask cuff 200 and establish a preliminary ventilation interface. The conduction portion 102 is configured for guiding and bearing internal channels. In this structure, the overall structure of the laryngeal mask is more reasonable, and various components can better work together, which not only ensures the functionality of the insertion end but also optimizes the passability and operability of the catheter portion.

Specifically, the conduction portion 102 in this embodiment adopts an arc-shaped structure to fit the natural physiological curvature of the human throat, making the path of the laryngeal mask from the mouth to the laryngeal position smoother and more natural, reducing unnecessary contact and resistance with oral and pharyngeal tissues, and making the insertion action easier to control.

Both the airflow channel 110 and the transmission channel 120 are integrated into the arc-shaped conduction portion 102, thereby ensuring the stability of gas transmission and avoiding entanglement and interference of external pipelines. It also enables the control position of the locking assembly 400 to be more centralized and in line with ergonomic principles. In this structure, it is ensured that the maintenance of the compressed state of the laryngeal mask during insertion is more reliable, and the opening operation of the locking assembly 400 after reaching the designated position is more convenient, thereby ensuring that the mask cuff 200 can form a stable and self-adaptive seal, and improving the final ventilation safety and sealing effect.

Combined with FIG. 2 and FIG. 3, in this embodiment, the laryngeal mask further includes an air source connector 500.

An air source port is formed at one end of the airflow channel 110 away from the mask cuff 200 and at one end of the conduction portion 102 away from the insertion portion 101, and a negative pressure port is formed at one end of the transmission channel 120 away from the mask cuff 200 and at the end of the conduction portion 102 away from the insertion portion 101. The air source connector 500 is connected with the air source port and configured to connect to an air source device. The negative pressure port is configured to connect with the locking assembly 400.

The air source connector 500 in this embodiment provides a standard and stable interface for connecting the ventilation device, ensuring the air tightness and reliability of the ventilation path connection. The negative pressure port is configured to connect with the locking assembly 400, making the structure configured for shape control more compact and centralized. In this structure, a doctor can firmly grip the conduction portion 102 with one hand during operation and conveniently operate the locking assembly 400 without worrying about accidentally touching the ventilation path.

Specifically, in this embodiment, the airflow channel 110 and the transmission channel 120 terminate at independent air source port and negative pressure port respectively at the ends away from the mask cuff 200, so that ventilation and compression/recovery have independent paths that do not interfere with each other. In this structure, connecting the air source device for ventilation and controlling the shape of the mask cuff 200 through the locking assembly 400 can be performed synchronously and independently, avoiding mutual coupling and potential influence between functions, so that the operation logic is clear.

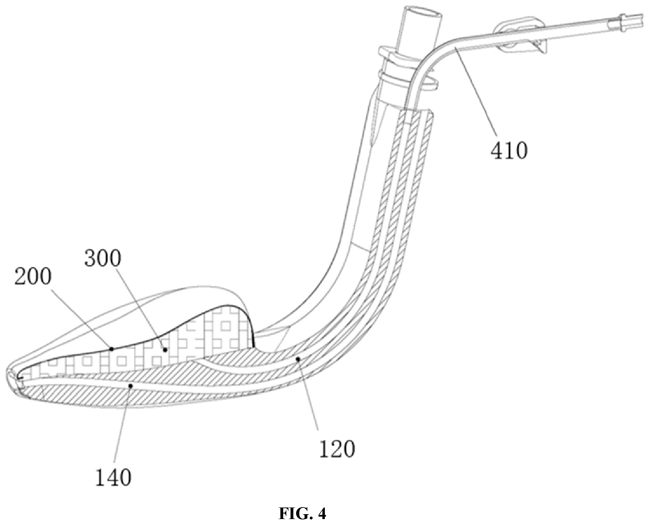

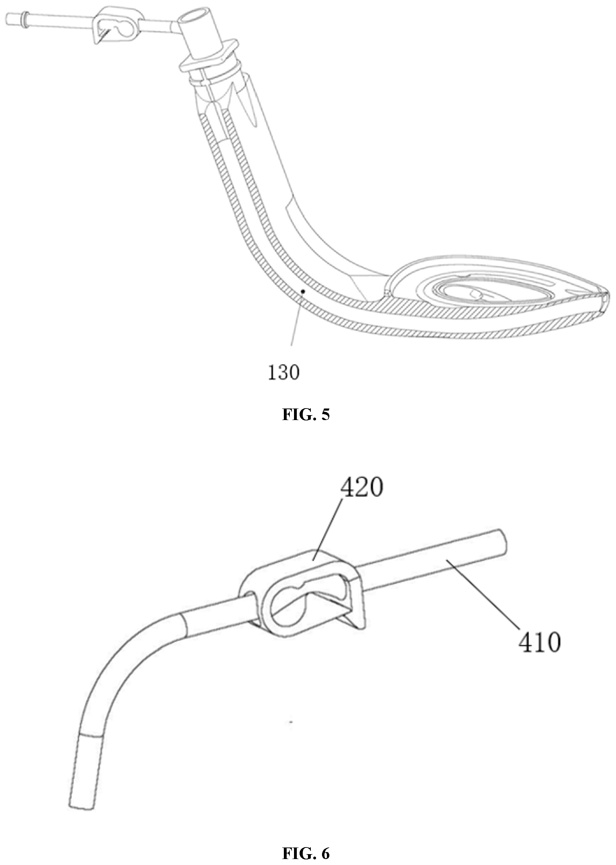

Please refer to FIG. 4 and FIG. 5 again. In this embodiment, a drainage channel 130 and an air intake channel 140 are further disposed in the laryngeal mask body 100.

One end of the insertion portion 101 away from the conduction portion 102 is provided with a drainage port. One end of the drainage channel 130 and one end of the air intake channel 140 are connected with the drainage port, and the other ends are respectively provided with a drainage tube port and an air intake port at the end of the conduction portion 102 away from the insertion portion 101.

Specifically, after the laryngeal mask is inserted to a preset position and fixed, a gastric tube can be inserted into the drainage channel 130, thereby providing a dedicated path for placing the gastric tube for gastrointestinal drainage. Gastric accumulated fluid or gas can be discharged through the gastric tube, thereby reducing the risk of reflux and aspiration of gastric contents during surgery. Meanwhile, the independent air intake channel 140 allows external air to enter during drainage, avoiding the formation of a closed negative pressure at the esophageal opening during the process of air pass. This not only prevents the esophageal mucosa from being adsorbed and injured, but also ensures the smoothness and safety of the drainage process.

The drainage channel 130, the air intake channel 140, the airflow channel 110, and the transmission channel 120, are all integrated within the insertion portion 101 and the arc-shaped conduction portion 102. In this structure, the smoothness of the overall outline of the laryngeal mask is ensured, avoiding affecting its insertion passability or operation feel due to externally connected additional pipelines, so that a doctor will not be interfered by extra pipelines when performing critical insertion and positioning operations.

Moreover, the drainage tube port and the air intake port in this embodiment are disposed at the end of the conduction portion 102 and spaced from the air source interface, realizing centralized management of multiple external functional interfaces. In this structure, it is convenient for medical personnel to quickly and orderly connect various functional pipelines (ventilation, drainage) after the laryngeal mask is placed, simplifying the subsequent operation flow and improving clinical efficiency.

Please refer to FIG. 6. In this embodiment, the locking assembly 400 includes an air pass tube 410 and a locking member 420.

The air pass tube 410 is inserted into the laryngeal mask body 100, and the transmission channel 120 is disposed in the air pass tube 410. The locking member 420 is disposed on the air pass tube 410, and the locking member is capable of closing the transmission channel 120.

When the laryngeal mask reaches the designated position of the throat, the operator only needs to open the closed channel through the locking member 420, and external air can rapidly enter the compression space via the air pass tube 410 and the transmission channel 120, so that the mask cuff 200 can timely and fully fit with the laryngeal anatomical structure, thereby achieving excellent dynamic sealing.

In this embodiment, the air pass tube 410 is a direct carrier of the transmission channel 120, and the air pass tube 410 is connected with the laryngeal mask body 100 in an inserting manner to form an airflow path. By operating the locking member 420 (such as a stop clip or a stopcock valve) disposed thereon, the transmission channel 120 can be reliably closed. This enables the compressed state of the mask cuff 200 to be firmly locked after manually pressing to discharge air, effectively preventing the unexpected expansion of the mask cuff 200 caused by air backflow during the insertion process, thereby ensuring that the laryngeal mask can pass through the throat curvature with the minimum volume and alleviating the problem of inconvenient insertion.

Optionally, in this embodiment, the locking member 420 is a stop clip. The stop clip is disposed on the air pass tube 410 and capable of clamping the air pass tube 410 to close the transmission channel 120.

The stop clip closes the transmission channel 120 by physically clamping the air pass tube 410 directly, which has high reliability and effectively ensures that the mask cuff 200 can stably maintain the compressed minimum volume state during the insertion process, avoiding accidental expansion caused by air leakage, thereby significantly reducing the resistance when passing through the throat curvature. Moreover, the stop clip is a mature medical device component in the prior art. The operation method (clamping and loosening) is simple and intuitive for medical personnel. In a surgical or emergency environment, all operations can be completed quickly and accurately with one hand, making the insertion of the laryngeal mask smoother and having high operation efficiency.

Optionally, the locking member 420 can further be a stopcock valve. The stopcock valve is installed on the air pass tube and disposed on the transmission channel 120. By rotating the valve core, the through hole inside the stopcock valve is aligned or misaligned with the flow channel of the valve body to open or close the transmission channel 120. The stopcock valve can place the transmission channel 120 in a fully open, fully closed, or partially open state, allowing for relatively precise graded adjustment of the flow rate. According to the material of the air pass tube 410, the stopcock valve and the air pass tube 410 can be connected by threaded connection, quick-plug connection, or flange connection, which is not limited herein. A sealing member, such as a sealing ring, can be disposed at the connection between the stopcock valve and the air pass tube to improve overall sealing performance.

In this embodiment, the laryngeal mask body 100 and the mask cuff 200 are adhesively connected to form a sealed structure at the connection.

The adhesive connection can ensure the air tightness of the connection, preventing gas in the compression space from leaking at the joint between the mask cuff 200 and the laryngeal mask body 100, thereby ensuring that an effective negative pressure can be formed in the compression space when extracting air through the negative pressure channel, so that the mask cuff 200 stably maintains the compressed state.

In addition, the adhesive connection can establish a firm mechanical combination between the laryngeal mask body 100 and the soft mask cuff 200, enabling the connection to withstand various stresses generated by the mask cuff 200 during repeated compression, rebound, and insertion processes, preventing detachment or tearing at the connection, thereby improving the structural strength and service life of the product and ensuring the functional stability of the laryngeal mask throughout the use process.

Optionally, the mask cuff 200 and the laryngeal mask body 100 can further be connected by thermal pressure welding. The connection area is precisely heated and pressurized to melt and fuse the contact surfaces of the mask cuff 200 and the compatible thermoplastic. This can form a homogeneous material transition zone without the brittleness of an adhesive layer, can better transmit and disperse stress, and withstand repeated compression and rebound. Moreover, it achieves a seamless seal and eliminates leakage.

In this embodiment, the mask cuff 200 is made of a polymer material.

Polymer materials such as medical-grade silicone or polyurethane have excellent elasticity and flexibility, enabling the mask cuff 200 to deform smoothly and reduce in volume under external pressing, and to recover smoothly to a predetermined shape under the drive of the internal resilient member 300 after pressure is released.

At the same time, medical-grade polymer materials have good biocompatibility and smooth surfaces, thereby reducing irritation and damage to the mucosal tissue of the throat and improving the clinical safety of the product.

In addition, polymer materials can be processed by mature injection molding or blow molding processes, facilitating precise control of the wall thickness uniformity and overall shape of the mask cuff 200, thereby ensuring the consistency and reliability of product performance.

It should be noted that to ensure safety of use, the Shore hardness of the material used for the mask cuff 200 should be less than 40, and a more appropriate selection is a Shore hardness between 4 and 12. In this embodiment, the mask cuff 200 is specifically made of medical silicone material with a Shore hardness of 10A.

Optionally, in this embodiment, the resilient member 300 is made of a porous elastic material.

The shape of the resilient member 300 is adapted to the compression space, so that the resilient member 300 fills the compression space.

Specifically, the interior of the porous elastic material contains a large number of interconnected pores, giving it extremely high compressibility. When extracting air through the negative pressure channel or manually pressing, the air inside the material is rapidly discharged, generating significant volume shrinkage.

After the pressure is released, the porous elastic material can rapidly suck in air through its elasticity and capillary action, recovering to its original shape filling the compression space. Moreover, the recovery deformation of the porous elastic material is not a simple shape memory, but a soft inward support capable of conforming to the surrounding structure, thereby driving the outer wall of the mask cuff 200 to dynamically and uniformly fit the unique laryngeal anatomical contours of different patients, forming a personalized sealing interface.

In addition, the resilient member 300 in this embodiment fills the entire compression space, thereby providing all-around, dead-angle-free uniform support for the mask cuff 200. In this structure, the solution can avoid situations of insufficient or excessive local support force, ensuring a stable distribution of sealing pressure. Furthermore, the porous structure also has advantages such as being lightweight and fatigue-resistant, guaranteeing the durability of the product.

The porous elastic material itself has flexible characteristics, so that the support of the resilient member 300 to the mask cuff 200 is "soft" rather than hard propping, thereby improving patient comfort and reducing the risk of mucosal injury.

Optionally, in this embodiment, the resilient member 300 is specifically medical sponge. Optionally, the resilient member 300 can also be made of a polymer foam material, such as polyurethane foam, which has good elasticity, is breathable and liquid-absorbent, can better fit the shape of the object being pressed, and distributes pressure evenly; or it can be rubber foam (such as neoprene, silicone foam, EPDM foam). Rubber foam usually has excellent sealing properties, aging resistance, ozone resistance, and a wider temperature range. Silicone foam also has excellent biocompatibility and chemical resistance.

The method of using for the laryngeal mask provided in this embodiment includes:
S100: compressing the mask cuff 200 and the resilient member 300 to discharge air in the compression space;
S200: adjusting the locking assembly 400 to close the negative pressure channel;
S300: applying a lubricant to the mask cuff 200 and one end of the laryngeal mask body 100 where the mask cuff 200 is provided;
S400: inserting the laryngeal mask body 100 into the mouth until the mask cuff 200 reaches a preset position; and
S500: adjusting the locking assembly 400 to conduct the negative pressure channel.

Specifically, steps S100 to S300 are preparation steps before insertion.

Before insertion, the mask cuff 200 and the resilient member 300 need to be actively compressed to the minimum volume to form a streamlined, compact front end, thereby reducing the contact area and frictional resistance between the laryngeal mask and surrounding tissues when passing through physiological curvatures such as the oral cavity and pharynx, solving the technical problems of "large volume and inconvenient insertion".

When the laryngeal mask reaches the preset position, by opening the locking assembly 400, external air can enter the compression space. The internal resilient member 300 immediately drives the mask cuff 200 to expand freely, rather than recovering into a preset fixed shape. The outer surface of the mask cuff 200 will gently and passively conform to the unique laryngeal anatomical contour of the patient, achieving a close fit with the throat and having excellent air tightness. Moreover, the operation process has clear logic and strong controllability, reducing dependence on the experience of the operator and improving the placement success rate and ventilation safety.

The preset position is the hypopharynx. The tip of the mask cuff 200 should be located above the upper esophageal sphincter (the opening of the esophagus), and both sides and the bottom of the mask cuff 200 need to completely and gently wrap the laryngeal inlet (including the epiglottis and glottis).

When the tip of the mask cuff 200 enters the hypopharynx and abuts against the upper esophageal sphincter, the operator will feel a clear, soft "resistance", indicating that the insertion depth is sufficient.

After releasing the mask cuff 200 (e.g., loosening the locking assembly 400), a slight but definite tendency for the entire laryngeal mask catheter to move outward can be observed or felt, indicating that the resilient member 300 is pushing the mask cuff 200 to fit closely with the laryngeal structure.

Furthermore, when finally confirming the position, manual ventilation can be performed on the airflow channel 110 by connecting an air source, while auscultating bilateral lung breath sounds and stomach gurgling sounds with a stethoscope, and observing chest rise and fall. If chest rise and fall are good, bilateral lung breath sounds are clear, and there is no stomach inflation sound, it is confirmed that the laryngeal mask is in an ideal "preset position".

Finally, it should be explained that the above embodiments are only used to illustrate the technical solutions of the present invention, not to limit them; although the present invention has been described in detail with reference to the foregoing embodiments, a person of ordinary skill in the art should understand: they can still modify the technical solutions described in the foregoing embodiments, or equivalently replace some or all of the technical features; however, these modifications or replacements do not cause the essence of the corresponding technical solutions to deviate from the scope of the technical solutions of the embodiments of the present invention.

### INDUSTRIAL APPLICABILITY

In summary, the embodiments of the present invention provide a laryngeal mask and a method of using therefor, which can solve the technical problems in the prior art that non-inflatable laryngeal masks have a large volume, are inconvenient to insert, and have poor adaptability, which may lead to poor sealing.

## Claims

1. A laryngeal mask, **characterized by** comprising:
a laryngeal mask body (100), wherein an airflow channel (110) is formed inside the laryngeal mask body (100);
a mask cuff (200), disposed at one end of the laryngeal mask body (100) and located at one side of the laryngeal mask body (100), wherein the mask cuff (200) is arranged along a circumferential direction of the laryngeal mask body (100) and encloses at the one side of the laryngeal mask body (100) to form a ventilation port communicating with the airflow channel (110), the mask cuff (200) is elastic and a compression space is formed therein;
a resilient member (300), disposed in the compression space and connected with an inner wall of the mask cuff (200); and
a locking assembly (400), having a negative pressure channel which communicates with the compression space and is openable and closable.

2. The laryngeal mask according to claim 1, wherein a transmission channel (120) is further formed in the laryngeal mask body (100);
one end of the transmission channel (120) is connected with the mask cuff (200) and communicates with the compression space, and the other end is connected with the locking assembly (400) and communicates with the negative pressure channel.

3. The laryngeal mask according to claim 2, wherein the laryngeal mask body (100) comprises: an insertion portion (101) and a conduction portion (102);
one side of the insertion portion (101) is an installation end provided with a first through hole communicating with the airflow channel (110), and the mask cuff (200) is disposed at the installation end; and
the conduction portion (102) is of an arc-shaped structure and one end of the conduction portion (102) is connected with the insertion portion (101), both the airflow channel (110) and the transmission channel (120) are disposed in the conduction portion (102), and the locking assembly (400) is connected with the conduction portion (102).

4. The laryngeal mask according to claim 3, wherein the laryngeal mask further comprises an air source connector (500);
an air source port is formed at one end of the airflow channel (110) away from the mask cuff (200) and at one end of the conduction portion (102) away from the insertion portion (101), and a negative pressure port is formed at one end of the transmission channel (120) away from the mask cuff (200) and at one end of the conduction portion (102) away from the insertion portion (101);
the air source connector (500) is connected with the air source port and configured to connect to an air source device; and
the negative pressure port is connected with the locking assembly (400).

5. The laryngeal mask according to claim 3 or 4, wherein a drainage channel (130) and an air intake channel (140) are further disposed in the laryngeal mask body (100);
one end of the insertion portion (101) away from the conduction portion (102) is provided with a drainage port; and
one end of the drainage channel (130) and one end of the air intake channel (140) are connected with the drainage port, and the other ends of the drainage channel (130) and the air intake channel (140) respectively form a drainage tube port and an air intake port at one end of the conduction portion (102) away from the insertion portion (101).

6. The laryngeal mask according to any one of claims 2 to 5, wherein the locking assembly (400) comprises: an air pass tube (410) and a locking member (420);
the air pass tube (410) is inserted in the laryngeal mask body (100), and the transmission channel (120) is disposed in the air pass tube (410); and
the locking member (420) is disposed on the air pass tube (410), and the locking member is capable of closing the transmission channel (120).

7. The laryngeal mask according to claim 6, wherein the locking member (420) is a stop clip; and
the stop clip is disposed on the air pass tube (410) and capable of clamping the air pass tube (410) to close the transmission channel (120).

8. The laryngeal mask according to any one of claims 1 to 7, wherein the laryngeal mask body (100) and the mask cuff (200) are adhesively connected to form a sealed structure at a connection.

9. The laryngeal mask according to any one of claims 1 to 8, wherein the resilient member (300) is made of a porous elastic material; and
a shape of the resilient member (300) is adapted to the compression space.

10. The laryngeal mask according to claim 9, wherein the resilient member (300) is a medical sponge.

11. The laryngeal mask according to any one of claims 1 to 10, wherein the mask cuff (200) is made of a polymer material.

12. The laryngeal mask according to claim 11, wherein a Shore hardness of the material of the mask cuff (200) is less than 40.

13. The laryngeal mask according to claim 12, wherein the Shore hardness of the material of the mask cuff (200) is 4 to 12.

14. The laryngeal mask according to any one of claims 11 to 13, wherein the mask cuff (200) is made of medical silicone or polyurethane material.

15. A method of using for a laryngeal mask, **characterized in that** the method of using is applied to the laryngeal mask according to any one of claims 1 to 14; and the method of using comprises:
compressing the mask cuff (200) and the resilient member (300) to discharge air in the compression space;
adjusting the locking assembly (400) to close the negative pressure channel;
applying a lubricant to the mask cuff (200) and the one end of the laryngeal mask body (100) where the mask cuff (200) is disposed;
inserting the laryngeal mask body (100) into a mouth until the mask cuff (200) reaches a preset position; and
adjusting the locking assembly (400) to conduct the negative pressure channel.
